# FASCICULE DE BREVET EUROPEEN

(11) **EP 0 784 993 B1**
(45) Date de publication et mention de la délivrance du brevet: **13.11.2002**
(21) Numéro de dépôt: 96402918.5
(22) Date de dépôt: 27.12.1996
(51) Int. Cl.: A61N 1/05

(54) **Sonde pour dispositif médical implanté, notamment pour stimulateur cardiaque**
Elektrodenzuleitung für implantierbare medizinische Vorrichtung, insbesondere für Herzschrittmacher
Electrode lead for implanted medical device, especially for heart pacemaker

(30) Priorité: 29.12.1995 FR 9515760
(43) Date de publication de la demande: 23.07.1997
(73) Titulaire: ELA MEDICAL (Société anonyme), F-92541 Montrouge Cédex (FR)
(72) Inventeur: Ollivier, Jean-Francois, 78280 Guyancourt (FR)
(74) Mandataire: Dupuis-Latour, Dominique

(56) Documents cités:
- EP-A- 0 546 414
- EP-A- 0 573 334
- US-A- 4 419 819
- US-A- 4 913 164
- US-A- 4 957 118

## Description

L'invention concerne les sondes pour dispositifs médicaux implantés, notamment pour stimulateurs cardiaques.

Ces sondes, notamment les sondes endocavitaires pour stimulateurs cardiaques, comportent des systèmes de rétention montés sur leur extrémité distale, à proximité de l'électrode de stimulation proprement dite (ou des électrodes dans le cas d'une sonde bipolaire).

Divers systèmes de rétention ou d'ancrage ont été proposés, notamment des fixations en forme de crochets ou "barbes" qui viennent se coincer d'elles-mêmes entre les trabéculations endocardiques.

Il peut être avantageux de concevoir ces systèmes d'ancrage de manière à autoriser, en cas de besoin, une explantation de la sonde sans risque d'arrachement des trabéculations ni des tissus fibreux formés autour de l'extrémité de la sonde.

Les systèmes dits "à fixation passive" sont conçus de manière que les barbes se logent d'elles-mêmes en place grâce à l'élasticité du matériau qui les constitue, leur forme inclinée assurant un effet anti-retour sans autre manoeuvre particulière.

Certaines fois il peut être nécessaire de procéder à l'explantation d'une sonde implantée depuis plusieurs années, en cas par exemple d'élévation du seuil de stimulation, d'infection ou encore en présence d'une sonde défectueuse pour cause de mauvaise isolation ou de rupture du conducteur.

Une première technique consiste à prévoir un actuateur mécanique, comme par exemple dans le US-A-4 957 118, où les barbes, initialement repliées contre le corps de la sonde, sont déployées en manoeuvrant un mandrin coaxial inséré dans la sonde au moment de l'implantation, et dont la rotation vient faire saillir les barbes une fois l'extrémité distale de la sonde positionnée à l'endroit voulu. Pour l'explantation de la sonde, les barbes sont repliées par une manoeuvre inverse du mandrin.

Ces sondes sont toutefois relativement encombrantes (le diamètre du corps est plus important car il faut prévoir un canal axial) et de structure plus complexe, dont plus coûteuses, du fait du système mécanique d'articulation et de déploiement des barbes et, d'autre part, de l'étanchéité qu'il faut assurer entre le système mécanique et le canal axial central de la sonde.

Pour permettre l'explantation sans risque d'arrachement des trabéculations ou des tissus fibreux, il est proposé dans le DE-A-31 46 182 de relier les barbes entre elles par un voile intermédiaire de manière à former une jupe continue et conique, l'ensemble étant réalisé en un élastomère de silicone ou de polyuréthanne. Au moment du retrait de la sonde, le cône s'inverse, évitant ainsi l'arrachement des tissus.

Cette sonde présente cependant un double inconvénient.

En premier lieu, la jupe conique, de par sa forme, empêche la croissance de la fibrose et procure donc, à long terme, un ancrage moins sûr que des barbes isolées.

En second lieu, le diamètre hors-tout de la sonde reste relativement important du fait de la forme évasée de la jupe, que ce soit à l'implantation ou à l'explantation : l'effet ressort de la jupe fait revenir le système, une fois détaché des trabéculations et des tissus, avec l'évasement du cône tourné en direction proximale, donc dans un état qui ne facilite pas le retrait de la sonde dans le circuit veineux. Du point de vue du diamètre, cette sonde passive ne procure aucun avantage particulier par rapport aux sondes à actuateur mécanique et impose donc les mêmes limitations au praticien lors de l'extraction de la sonde à travers les voies veineuses qui ont été empruntées lors de la mise en place de celle-ci.

Au surplus, l'extraction d'une telle sonde, qui provoque généralement un arrachement des tissus fibreux, entraîne une surépaisseur en extrémité du fait de la présence de ces tissus, qui vont former un bouchon représentant un obstacle considérable au retrait.

Une autre sonde de ce type est décrit dans le document US-A-4 913 164.

L'un des buts de la présente invention est de proposer une sonde du type à "fixation passive" précité, qui puisse être mise en place sans actuateur, qui présente un faible diamètre à l'extraction, qui soit de structure simple et donc peu coûteuse et qui permette une explantation aisée sans endommagement des tissus de la cavité endocardique ni des parois veineuses.

À cet effet, la sonde de l'invention, qui est du type comportant à son extrémité un corps cylindrique portant l'électrode ou les électrodes de la sonde et pourvu de moyens d'ancrage faisant saillie radialement vers l'extérieur, comporte un manchon déformable en matériau souple gainant le corps, ce manchon étant, dans une partie de liaison, solidarisé au corps à l'extrémité distale de ce dernier et, dans une partie libre s'étendant proximalement par rapport à la partie de liaison et portant les moyens d'ancrage, essentiellement appliqué de manière séparable contre le corps sur le reste de ladite région distale.

Selon des caractéristiques subsidiaires avantageuses :
- le matériau du manchon est choisi de manière à autoriser un retournement du manchon en cas de sollicitation exercée axialement dans le sens distal sur les moyens d'ancrage, le manchon en position retournée étant tel que la partie libre, avec les moyens tournés radialement vers l'intérieur, s'étende distalement par rapport à la partie de liaison ; dans ce cas, la position retournée est avantageusement un état mécaniquement stable, et la surface externe du manchon à l'état retourné est de préférence une surface essentiellement lisse, dépourvue d'éléments saillants ;
- les moyens d'ancrage comportent une pluralité de barbes d'ancrage ;
- le manchon et les barbes sont constitués par un élément moulé monobloc ;
- la partie de liaison comporte une partie annulaire enserrée entre l'électrode côté distal et une face frontale du corps côté proximal ;
- partie de liaison et partie libre sont reliées par une région d'épaisseur réduite formant charnière au moment du retournement du manchon ;
- la sonde comportant une gaine isolante prolongée par le corps portant l'électrode, la longueur de la partie libre du manchon en direction proximale est choisie de manière à recouvrir entièrement le corps et déborder sur la gaine adjacente, en contact d'étanchéité avec celle-ci. ;
- il est prévu un agent lubrifiant appliqué entre le corps et la partie libre du manchon ;
- le manchon est en matériau radio-opaque.

D'autres caractéristiques et avantages de l'invention apparaîtront à la lecture de la description détaillée ci-dessous, faite en référence aux dessins annexés.

La figure 1 est une vue en coupe de l'extrémité distale d'une sonde selon l'invention, la demi-coupe supérieure montrant la configuration de la sonde dans sa position d'insertion et d'ancrage et la demi-coupe inférieure montrant la configuration de la sonde au début de la phase de retrait.

La figure 2 est une coupe de l'extrémité distale de la sonde de l'invention dans une phase ultérieure du retrait, lorsque la sonde se détache des tissus environnants.

Sur les figures, la référence 10 désigne, de façon générale, l'extrémité distale de la sonde. Cette dernière est, sur l'exemple illustré, une sonde monopolaire comportant une gaine isolante souple 12 prolongée par un élément cylindrique rigide 14 portant sur sa face frontale (région distale) l'électrode proprement dite 16. Cette électrode comporte une tête aplatie 18, destinée à venir en contact avec la paroi du myocarde, prolongée intérieurement par une tige 20 assurant la liaison avec le conducteur 22 ; ce conducteur est en forme de ressort spiralé se poursuivant à l'intérieur de la gaine souple 12 jusqu'à l'extrémité proximale de la sonde, reliée au stimulateur. La réalisation de cette partie de la sonde peut notamment être comparable à celle décrit dans le EP-A-0 296 001 au nom d'ELA Médical.

De façon caractéristique de l'invention, la partie terminale rigide 14 du corps de sonde est recouverte d'un manchon 24 qui vient la gainer. Ce manchon 24 comporte, d'une part, une partie de liaison annulaire 26 pincée entre la face distale de l'élément 14 et la partie aplatie 18 de l'électrode, et, d'autre part, une partie libre 28 recouvrant l'élément 14 (par "libre", on signifie que cette partie 28 est appliquée contre l'élément 14 constituant le corps de sonde, mais n'y est pas solidarisée, cette solidarisation n'étant effective que dans la partie de liaison 26).

En direction proximale, la partie libre 28 est prolongée jusqu'en 30, en recouvrement de l'extrémité distale 32 de la gaine 12, qui est enserrée à cet endroit entre, d'une part, le corps de sonde et, d'autre part, la partie libre du manchon 28. Pour éviter une surépaisseur, l'extrémité distale de la gaine 12 et/ou l'extrémité proximale du manchon 28 peuvent présenter une épaisseur amoindrie à l'endroit de leur recouvrement. D'autre part une certaine étanchéité peut être obtenue par une constriction diamétrale de la partie libre à son extrémité proximale 30, de façon que cette partie libre adhère le plus possible au corps 14 et à la gaine souple 12, avec adhérence silicone sur silicone à ce dernier endroit.

Le manchon 28 porte, de manière en elle-même connue, une pluralité de barbes d'ancrage distinctes 34 qui vont venir se loger entre les trabéculations (telles que représentées en 36) de la cavité endocardique, la forme inclinée en direction proximale assurant un effet anti-retour ; en outre ces barbes seront enveloppées à la longue par une fibrose, venant assujettir d'autant plus fermement en place l'extrémité de sonde du fait de la forme saillante de ces barbes.

Avantageusement, la région 38 assurant la transition entre la partie libre 28 et la partie de liaison 26 est une région amincie, de manière à procurer un effet de charnière entre ces deux parties.

Le manchon est en un matériau souple, par exemple un élastomère de silicone (tel qu'un ETRQ7 47.80 d'une dureté Shore de l'ordre de 30 à 80) présentant une souplesse suffisante pour autoriser les déformations qui seront décrites par la suite. Ce matériau ne doit cependant pas être trop souple, car il doit résister au retroussement lors des essais répétés à l'implantation, lorsque le praticien imprime à la tête de sonde un mouvement d'avance et de recul pour réaliser l'imbrication dans les trabéculations.

L'introduction d'une telle sonde est en elle-même classique, et comparable à celle d'une sonde à fixation passive de type connu.

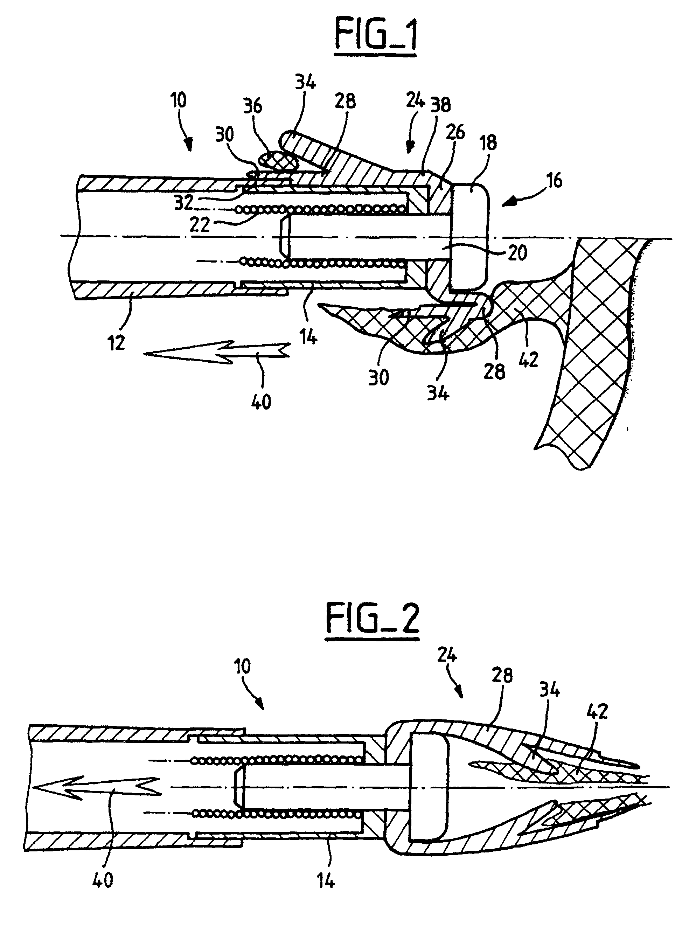

L'intérêt de la sonde se révèle au moment de l'explantation définitive : lorsque le praticien exerce une traction en direction proximale (flèche 40), la partie libre 28, retenue par la fibrose 42 qui est venue se former autour des barbes 34, va glisser sur l'élément rigide 14 du corps de sonde et s'en détacher progressivement, comme illustré sur la demi-coupe inférieure de la figure 1.

Ce glissement peut être avantageusement favorisé par adjonction d'un fluide ou d'une pâte lubrifiante entre la face extérieure de l'élément 14 et la face intérieure de la partie libre 28 du manchon 24.

L'ensemble de ces actions va provoquer un retroussement du manchon autour de la zone charnière 38, aboutissant à la configuration illustrée figure 2, c'est-à-dire avec la partie libre 28 en arrière (dans le sens de l'extraction) du corps de sonde 14 et les barbes 34 tournées vers l'intérieur de la partie libre retroussée.

On notera que la position retroussée illustrée figure 2 constitue un état mécanique stable, qui permettra le retrait aisé de la sonde dans le circuit veineux, la surface extérieure du manchon retroussée offrant une surface essentiellement lisse, puisque les barbes 34 sont maintenant tournées vers l'intérieur.

À ce stade, deux cas de figure sont possibles.

Dans le premier cas, la fibrose vient se détacher d'elle-même des barbes et reste accrochée au myocarde. Le début du processus a cependant permis de retrousser le manchon, et les barbes sont donc dirigées vers l'intérieur et ne s'opposent pas au retrait lors du parcours dans les veines ; on se trouve dans la même configuration que celle que l'on aurait eue avec une sonde lisse, "monodiamètre". La séparation des barbes d'avec la fibrose est favorisée si l'on donne à ces barbes une forme légèrement effilée, c'est-à-dire si leur dimension en section transversale est plus faible près de leur extrémité que près de leur racine.

Dans le second cas de figure, la fibrose continue, partiellement ou totalement, à adhérer à la partie libre du manchon, et va donc se déchirer au cours du processus d'extraction. Cependant, comme on peut le voir sur la figure 2, les fibres arrachées vont se trouver en arrière (dans le sens de l'extraction) de la tête de sonde et ne vont pas créer de surépaisseur ou de bouchon risquant de bloquer l'extraction lors du trajet intraveineux.

Avantageusement, le manchon peut être réalisé en un matériau chargé de particules radio-opaques pour permettre de suivre le déroulement du processus d'extraction sous amplificateur de brillance.

L'explantation se poursuit jusqu'à l'extraction complète de la sonde. On notera que la structure de l'invention permet de retirer la totalité de la sonde, ce qui est un avantage majeur lorsque le patient est sujet à une infection généralisée. D'autre part, l'extraction ne nécessite aucun outil spécifique tel que gaine de découpe, extracteur de Cook, etc., qui sont parfois difficiles à introduire dans la sonde ou autour de celle-ci.

## Revendications

1. Une sonde (10) pour dispositif médical implanté, notamment pour stimulateur cardiaque, comportant à son extrémité un corps cylindrique (14) portant l'électrode (18) ou les électrodes de la sonde et pourvu de moyens d'ancrage (34) faisant saillie radialement vers l'extérieur,
**caractérisée en ce qu'**elle comporte un manchon déformable (24) en matériau souple gainant le corps, ce manchon étant, dans une partie de liaison (26), solidarisé au corps à l'extrémité distale de ce dernier et, dans une partie libre (28) s'étendant proximalement par rapport à la partie de liaison et portant les moyens d'ancrage, essentiellement appliqué de manière séparable contre le corps sur le reste de ladite région distale.

2. La sonde de la revendication 1, dans laquelle le matériau du manchon est choisi de manière à autoriser un retournement du manchon en cas de sollicitation (40) exercée axialement dans le sens distal sur les moyens d'ancrage, le manchon en position retournée étant tel que la partie libre, avec les moyens tournés radialement vers l'intérieur, s'étende distalement par rapport à la partie de liaison.

3. La sonde de la revendication 1, dans laquelle les moyens d'ancrage comportent une pluralité de barbes d'ancrage (34).

4. La sonde de la revendication 2, dans laquelle la position retournée est un état mécaniquement stable du manchon.

5. La sonde de la revendication 2, dans laquelle la surface externe du manchon à l'état retourné est une surface essentiellement lisse, dépourvue d'éléments saillants.

6. La sonde de la revendication 1, dans laquelle le manchon et les moyens d'ancrage sont constitués par un élément moulé monobloc.

7. La sonde de la revendication 1, dans laquelle la partie de liaison comporte une partie annulaire enserrée entre l'électrode côté distal et une face frontale du corps côté proximal.

8. La sonde de la revendication 1, dans laquelle partie de liaison et partie libre sont reliées par une région d'épaisseur réduite (38) formant charnière au moment du retournement du manchon.

9. La sonde de la revendication 1, dans laquelle, la sonde comportant une gaine isolante (12) prolongée par le corps portant l'électrode, la longueur de la partie libre du manchon en direction proximale est choisie de manière à recouvrir entièrement le corps et déborder sur la gaine adjacente, en contact d'étanchéité avec celle-ci.

10. La sonde de la revendication 1, dans laquelle il est prévu un agent lubrifiant appliqué entre le corps et la partie libre du manchon.

11. La sonde de la revendication 1, dans laquelle le manchon est en matériau radio-opaque.

## Claims

1. A lead (10) for an implanted medical device, in particular a cardiac pacemaker, comprising at it end a cylindrical body (14) that carries the electrode (18) or electrodes of the lead and provided with anchoring means (34) that project radially outwards,
**characterised in that**
it comprises a deformable sleeve (24) made of a flexible material sheathing the body, the said sleeve being attached at a connection portion (26) to the body at the distal end of the latter and, in a free portion (28), extending proximally relative to the connection portion and carrying the anchoring means, essentially applied in a separable way against the body over the remainder of the said distal region.

2. The lead of Claim 1, in which the sleeve material is chosen such as to enable the sleeve to turn back upon itself in the event that a force (40) is exerted axially on the anchoring means in the distal direction, the sleeve in its turned-back position being such that the free portion, with its means turned back radially inwards, extends distally relative to the connection portion.

3. The lead of Claim 1, in which the anchoring means comprise a plurality of anchoring barbs (34).

4. The lead of Claim 2, in which the turned-back position is a mechanically stable condition of the sleeve.

5. The lead of Claim 2, in which the outer surface of the sleeve in the turned-back condition is an essentially smooth surface having no projecting elements.

6. The lead of Claim 1, in which the sleeve and the anchoring means consist of a moulded one-piece element.

7. The lead of Claim 1, in which the connection portion comprises an annular part held between the electrode on the distal side and a front face of the body on the proximal side.

8. The lead of Claim 1, in which the connection portion and the free portion are joined by a region of smaller thickness (38) that acts as a hinge when the sleeve is turned back.

9. The lead of Claim 1, in which the lead comprises an insulating sheath (12) extended by the body carrying the electrode, and the length of the free portion of the sleeve in the proximal direction is chosen so as entirely to cover the body and overlap over the adjacent sheath, in leakproof contact with the latter.

10. The lead of Claim 1, in which a lubricant is provided, applied between the body and the free portion of the sleeve.

11. The lead of Claim 1, in which the sleeve is made of a radio-opaque material.

## Patentansprüche

1. Fühler (10) für eine medizinische, implantierte Vorrichtung, insbesondere für einen Herzschrittmacher, welcher an seinem Ende einen zylindrischen Körper (14) aufweist, der die Elektrode (18) oder die Elektroden des Fühlers trägt und mit Verankerungsmitteln (34) versehen ist, welche radial in Richtung nach außen vorragen, **dadurch gekennzeichnet, dass** er eine deformierbare Muffe (24) aufweist aus nachgiebigem Material, die den Körper umhüllt, wobei diese Muffe in einem Verbindungsteil (26) mit dem Körper am fernen Ende dieses Letzteren verbunden ist und sich in einem freien Teil (28) nahe in Bezug auf den Verbindungsteil erstreckt und die Verankerungsmittel trägt, die im Wesentlichen auf trennbare Weise gegen den Körper auf dem Rest des entfernten Bereichs angelegt sind.

2. Fühler nach Anspruch 1, bei dem das Material der Muffe in einer Weise ausgewählt ist, um ein Umschlagen der Muffe im Fall einer Belastung (40) zu erlauben, welche axial in der entfernten Richtung auf die Verankerungsmittel ausgeübt wird, wobei die Muffe in umgeschlagener Position derart beschaffen ist, dass der freie Teil sich mit den radial umgekehrten Mitteln in Richtung nach innen entfernt in Bezug auf den Verbindungsteil erstreckt.

3. Fühler nach Anspruch 1, bei welchem die Verankerungsmittel eine Mehrzahl von Verankerungswiderhaken (34) aufweisen.

4. Fühler nach Anspruch 2, bei welchem die umgeschlagene Position ein mechanisch stabiler Zustand der Muffe ist.

5. Fühler nach Anspruch 2, bei welchem die äußere Oberfläche der Muffe in umgeschlagenem Zustand eine im Wesentlichen glatte Oberfläche ist, ohne vorragende Elemente.

6. Fühler nach Anspruch 1, bei welchem die Muffe und die Verankerungsmittel durch ein gegossenes, einstückiges Element gebildet werden.

7. Fühler nach Anspruch 1, bei welchem der Verbindungsteil einen ringförmigen Teil aufweist, der zwischen der Elektrode der fernen Seite und einer Stirnfläche des Körpers der nahen Seite eingespannt ist.

8. Fühler nach Anspruch 1, bei welchem der Verbindungsteil und der freie Teil durch einen Bereich reduzierter Dicke (38) verbunden sind, welcher im Moment des Umschlagens der Muffe ein Schatnier bildet.

9. Fühler nach Anspruch 1, bei welchem der Fühler eine Isolierhülle (12) aufweist, welche durch den Körper, der die Elektrode trägt, verlängert ist, wobei die Länge des freien Teils der Hülse in naher Richtung in einer Weise gewählt ist, um vollständig den Körper abzudecken und über die benachbarte Hülle in dichtem Kontakt mit dieser herüberragt.

10. Fühler nach Anspruch 1, bei welchem ein Schmiermittel vorgesehen ist, das zwischen dem Körper und dem freien Teil der Hülse appliziert ist.

11. Fühler nach Anspruch 1, bei welchem die Hülse aus einem Funkundurchlässigem Material ist.
